# EUROPEAN PATENT APPLICATION

(11) **EP 2 277 479 A1**
(43) Date of publication of application: **26.01.2011**
(21) Application number: 10169782.9
(22) Date of filing: 16.07.2010
(51) Int. Cl.: A61F 5/08, A61F 5/56

(54) **Nasal device designed to prevent snoring and sleep apnea**

(30) Priority: 24.07.2009 IT VR20090116
(71) Applicant: Perbellini, Fabiano, 37060 Castel d'Azzano (VR) (IT)
(72) Inventor: Perbellini, Fabiano, 37060 Castel d'Azzano (VR) (IT)
(74) Representative: Sandri, Sandro

(57) **Abstract**

A nasal device designed to prevent the phenomenon of snoring and apnea during sleep by inserting the device into the nostrils, and where the device (10) substantially comprises two or more rings (11, 12), joined crossways to each other.

## Description

### TECHNICAL FIELD

The present invention relates to a nasal device designed to prevent snoring and sleep apnea.

In particular, the present invention refers to an anti-snoring nasal device substantially comprising two, three or more rings joined crossways to each other to form a type of nasal divaricator designed to hold the nostrils open.

The nasal device according to the present invention is simply inserted into the nasal cavities and enables the complete elimination of sleep apnea and eliminates or at least drastically reduces the phenomenon of snoring with all the advantages that result from this reduction.

The present invention applies to the sector for medical and paramedical devices designed to alleviate the various forms of snoring.

### BACKGROUND ART

It is well known that the phenomenon of snoring is caused in humans by the passage of air through a narrowing of the upper airways.

There are various remedies for the phenomenon of snoring. Remedies include treatment with pharmaceuticals which assist improvements in breathing through broncho constriction and dilatation; this remedy is suitable for light cases and does not appear to provide a complete cure. Treatments involving mechanical ventilators range from controlled ventilation proper (reserved for hospitalised patients) to ventilation masks for home use which are simple and practical to use. Surgical treatment is reserved for those cases where an endoscopic examination of the nose, throat and bronchial tree reveals the effective presence of an obstruction to be removed.

There are also other mechanical devices commercially available such as nasal patches applied to the outside of the nose and kept in place by adhesive material.

Nasal patches of this type have a rib which allows the nostrils to remain open thus improving the flow of air thereby alleviating nasal congestion and helping to reduce snoring.

These patches help to improve the quality of sleep of persons suffering from snoring problems and provide some relief without the need to resort to pharmaceuticals.

However, it has been found that these patches do not cure snoring problems completely since their action in keeping the nostrils open is limited. They also cause a certain amount of irritation in the user since the patches are fixed with an adhesive material which can cause irritation and in general are not liked by potential users.

### DESCRIPTION OF THE INVENTION

The present invention provides an anti-snoring nasal device designed to prevent sleep apnea and which eliminates or at least reduces the shortcomings described above.

The invention also provide an anti-snoring nasal device which is simple to make using very simple means and can therefore be distributed on a large-scale.

This is achieved by means of an anti-snoring nasal device designed to prevent sleep apnea whose characteristics are described in the main claim.

The dependent claims of the present invention describe advantageous embodiments of the invention.

The main advantages of this solution, in addition to those advantages deriving from its simple construction, concern the fact that the anti-snoring nasal device can be inserted in the nostrils without causing any irritation and with good results given that the present invention provides a non-invasive way of opening the nostrils.

The device according to the present invention substantially comprises the joining together of two, or three, or multiple rings welded to each other crossway at approximately 90° or 60° and which comprise a type of nasal divaricator which holds the nostrils open.

The rings which comprise the nasal device according to the present invention are suitably dimensioned and used by simply inserting each device into the nasal cavities so as to eliminate or at least drastically reduce sleep apnea and the phenomenon of snoring in general with all the advantages which result from this.

It should be noted that the object can be made by welding together two, three or multiple rings and also by making a single casting to obtain a one-piece component.

### BRIEF DESCRIPTION OF THE DRAWINGS

Further features and advantages of the invention will become apparent from the description of an example embodiment which follows with reference to the annexed drawings, given purely by way of a non-limiting example, in which:
- Figure 1 shows a diagram of one of the nasal devices according to the present invention in its entirety;
- Figure 2 shows a diagram of one of the nasal devices according to the invention as seen from another angle;
- Figure 3 is a diagram simulating the insertion into one of the nasal cavities of one of the nasal devices according to the invention;
- Figure 4 shows a diagram of the nasal device according to the invention in a version with three rings joined together.

### DESCRIPTION OF AN EXAMPLE EMBODIMENT

With reference to the annexed drawings, the nasal device according to the invention designed to prevent the phenomenon of snoring and to prevent apnea during sleep is indicated in its entirety by the numeral 10 and substantially comprises a pair of rings 11 and 12, welded crossways to each other.

In particular, the nasal device according to the invention where one device is inserted into one of the two nasal cavities, or nostrils, while another identical device is inserted into the other nasal cavity, comprises two or three rings welded together to form a cross of circular elements at 90° to each other or at 60° to each other in the case of the three-ring version.

The device forms a type of framework which has a substantially spherical shape.

The rings 11 and 12 are welded to each other at the crossovers, that is, close to the zones 13 and 14, where the rings make contact with each other and are fixed to each other exactly at these points.

It should be noted that the object can be made not only by welding together two or three rings but also by making a single casting to obtain a one-piece component.

A construction of this type provides a type of nasal divaricator designed to hold the nostrils wide open during sleep.

Use of the nasal device according to the present invention involves simply inserting them into the nasal cavities, that is, one for each nostril, without exerting any pressure.

In particular the devices are inserted manually in both nostrils and pushed upwards until they meet the first narrowing consisting of the middle nasal concha located in the upper part of the nasal fossa.

The position of the crossed rings inserted inside the nostrils assists the dilatation of the nostrils allowing a greater passage of air and thereby eliminating nocturnal apnea and drastically reducing or even eliminating the phenomenon of snoring with all the advantages which this brings in terms of improved sleep and rest in general.

Clearly, numerous and various modifications can be made to the device described. It can also be made from numerous and various different types of material. Non-oxidising metals such as gold, stainless steel, silver and titanium are recommended but more economic materials such as plastic, rubber, resin or similar can also be used. Devices made from plastic rubber and resin have the advantage that they can be thrown away after use thereby avoiding the risk of bacteria or other phenomenon which could cause nasal irritation if the device were to be reused.

It is also foreseen that the devices described could be made in pairs, so that there is one device for each nostril, and that each pair of nasal devices could be marketed in various sizes and dimensions to match the dimensions of the user's nasal cavities.

It is also foreseen that the crossed rings could have any shape. They could be circular, elliptic or have any other suitable shape. It is also foreseen that the wires comprising the rings could have a circular, flat or semi-circular cross section.

Advantageously it is also foreseen that, in the interests of safety, a pair of devices 10 to be inserted into the two nostrils can be attached to each other with a wire or a small chain in order to keep them together as a pair.

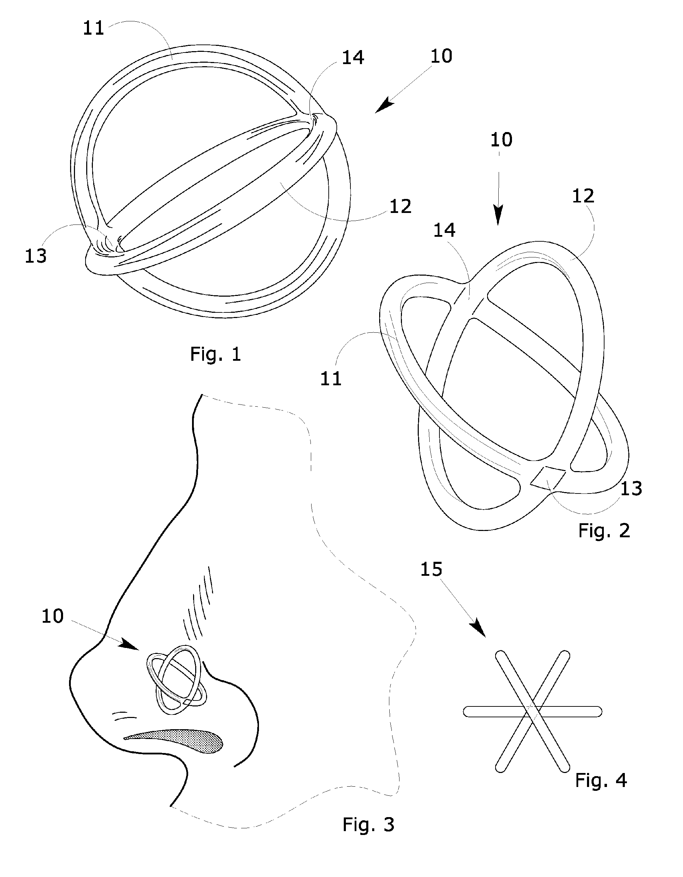

As Figure 4 shows, the rings 15 joined together could be more than two; for example they could be three joined together at 60°, or could be multiple rings arranged to occupy other various spaces.

The invention as described above refers to a preferred embodiment. Naturally, while the principle of the invention remains the same, the details of construction and the embodiments may vary widely with respect to what has been described and illustrated purely by way of example, without departing from the scope of the present invention.

## Claims

1. A nasal device designed to prevent the phenomenon of snoring and designed to prevent apnea during sleep by inserting the device into the nostrils, **characterised in that** the device (10) substantially comprises two or three rings (11, 12), with a circular, elliptic or similar shape, joined to each other by welding or also made as a single casting to obtain a one-piece component.

2. The nasal device according to the foregoing claim, **characterised in that** the device (10), comprising two or three crossed rings, can be inserted into each of the two nasal cavities, or nostrils, and also **characterised in that** the pair of rings are welded together to form a cross of components which are substantially of a circular, elliptical or similar shape, at approximately 60°/90° to each other, or can be made as a single casting to obtain a one-piece component.

3. A nasal device according to one of the foregoing claims, **characterised in that** the arrangement of the rings forming the device (10) form a type of framework which has a substantially spherical shape designed to keep the nostrils wide open when inserted in the nostrils.

4. A nasal device according to one of the foregoing claims, **characterised in that** the rings (11, 12) are welded to each other at the crossovers, that is, close to the zones (13, 14) where the rings make contact with each other and are fixed to each other exactly at these points.

5. A nasal device according to one of the foregoing claims, **characterised in that** the rings (11, 12) are made as a single casting by hot moulding or a similar method.

6. A nasal device according to one of the foregoing claims, **characterised in that** it comprises a type of nasal divaricator designed to hold the nostrils wide open during sleep.

7. A nasal device according to one of the foregoing claims, **characterised in that** the devices are inserted manually in both nostrils and pushed upwards until they meet the first narrowing comprising the internal, middle nasal sector in the upper part of the nasal fossa.

8. A nasal device according to one of the foregoing claims, **characterised in that** the crossed rings can have a circular, elliptical or any other suitable shape and that the cross-section of the wires comprising the rings can be circular, flat, semi-circular or similar.

9. A nasal device according to one of the foregoing claims, **characterised in that** for the safety of the devices it is foreseen that the pair of devices (10) to be inserted into the two nostrils, can be attached to each other with a wire or a small chain in order to keep them together as a pair.

10. A nasal device according to one of the foregoing claims, **characterised in that** the circular, elliptical or similarly shaped rings joined to each other can be more than two, for example three joined together 60°, or could be multiple rings arranged to occupy other spaces.
